# Europäisches Patentamt

(19) **European Patent Office**

Office européen des brevets

(11) Publication number: **0 034 054**
**B1**

(12) # EUROPEAN PATENT SPECIFICATION

(45) Date of publication of the patent specification:
02.02.83

(51) Int. Cl.³: **C 07 C 7/13,** C 07 C 15/067,
B 01 J 20/18

(21) Application number: **81300504.8**

(22) Date of filing: **06.02.81**

(54) Process for the separation of three components from a mixture by solid-bed adsorption and desorption.

(30) Priority: **07.02.80 US 123227**
**28.04.80 US 144757**

(43) Date of publication of application:
**19.08.81 Bulletin 81/33**

(45) Publication of the grant of the patent:
**02.02.83 Bulletin 83/5**

(84) Designated Contracting States:
**DE FR GB IT NL**

(56) References cited:

**US-A-3 732 325**
**US-A-4 028 428**
**US-A-4 031 156**
**US-A-4 079 094**
**US-A-4 108 915**

(73) Proprietor: **UOP Inc., 10 UOP Plaza Algonquin & Mt.**
**Prospect Roads, Des Plaines Illinois 60016 (US)**

(72) Inventor: **Broughton, Donald Bedoes, 1639 Hinman**
**Avenue, Evanston Illinois (US)**

(74) Representative: **Hale, Stephen Geoffrey et al, J.Y. &**
**G.W. Johnson Furnival House 14/18 High Holborn,**
**London WC1V 6DE (GB)**

# Process for the separation of three components from a mixture by solid-bed adsorption and desorption

The present invention relates to a solid-bed adsorptive separation process for separating three components from a mixture thereof utilizing an adsorbent-desorbent combination exhibiting the desired selectivity for each of such components.

It is well known in the separation art that certain crystalline aluminosilicates can be used to separate one hydrocarbon type from another hydrocarbon type. The separation of normal paraffins from branched chained paraffins, for example, can be accomplished by using a type A zeolite which has pore openings from 3 to 5 Angstroms. Such a separation process is disclosed in U. S. Patents 2,985,589 and 3,201,491. These adsorbents allow a separation based on the physical size difference in the molecules by allowing the smaller, normal hydrocarbons to be passed into the cavities within the zeolitic adsorbent, while excluding the larger, branched chain molecules. U. S. Patents 3,265,750 and 3,510,423, for example, disclose processes in which larger pore diameter zeolites such as the type X or type Y zeolites can be used to separate olefinic hydrocarbons from non-olefinic hydrocarbons.

In addition to being used in processes for separating hydrocarbon types, adsorbents comprising type X or Y zeolites have also been employed in processes for separating individual hydrocarbon isomers. In the processes described in U. S. Patents 3,558,730; 3,558,732; 3,626,020 and 3,686,342, for example, particular zeolitic adsorbents are used to separate desired xylene isomers; in U. S. Patent 3,114,782 they are used to separate alkyl-trisubstituted benzenes: in U. S. Patent 3,864,416 they are used to separate tetraalkyl substituted aromatic hydrocarbon isomers; and in U. S. Patent 3,668,267 they are used to separate particular alkyl substituted naphthalenes.

The principle of using a simulated moving bed of adsorbent for the continuous separation of the components of a fluid mixture by contact with a solid adsorbent is set forth in U. S. Patent 2,985,589. The movement of the adsorbent bed is simulated by periodically advancing through the column of adsorbent the various points of introducing and withdrawing the liquid streams.

A common problem with the simulated moving bed flow scheme is that the raffinate itself contains components the separation of which is desired, but which cannot be separated by conventional means, such as fractionation. In such cases, therefore, it has been the practice to provide a second-stage simulated moving bed to achieve the complete separation. A method of operating a simulated moving bed column has now been discovered which avoids the aforementioned problem that would otherwise occur with certain three-component feedstocks.

The process is particularly useful in separating ethylbenzene and ortho-, meta- and para-xylene from mixtures in which they occur.

According to the invention there is provided a process for separating first, second and third components from a fluid feed stream comprising a mixture of said components employing an adsorbent and a desorbent, which process comprises the steps of:

a) maintaining net fluid flow in a single direction through a column of the adsorbent containing at least three zones having separate operational functions occurring therein and being serially interconnected to provide a continuous cycle;

b) maintaining in the column an adsorption zone comprising the adsorbent located between a feed input at its upstream boundary and a raffinate output at its downstream boundary;

c) maintaining a purification zone in the column immediately upstream from the adsorption zone and comprising the adsorbent located between an extract output at its upstream boundary and the feed input at its downstream boundary;

d) maintaining a desorption zone in the column immediately upstream from the purification zone and comprising the adsorbent located between a desorbent input at its upstream boundary and the extract output at its downstream boundary;

e) passing the feed stream via the feed input into the adsorption zone at adsorption conditions to effect the selective adsorption of the frist component by the adsorbent in the adsorption zone;

f) passing a desorbent via the desorbent input into the desorption zone at desorption conditions to effect the displacement of the first component from the adsorbent in the desorption zone;

g) withdrawing an extract stream comprising the first component and desorbent material from the desorption zone via the extract output;

h) withdrawing a raffinate stream comprising a component of the fluid feed stream and desorbent material from the adsorption zone via the raffinate output; and

i) periodically advancing through the column of adsorbent, in a downstream direction with respect to fluid flow, the feed input, raffinate output, desorbent input and extract output to effect the shifting of zones through the adsorbent;

characterized in that the adsorbent and desorbent in combination exhibit relative selectivities for the first, second and third components respectively in decreasing orders of magnitude; that the withdrawn raffinate stream comprises the third component of the fluid feed stream and desorbent; that an intermediate raffinate stream comprising a mixture of the second component, the third component and desorbent is withdrawn from the adsorption zone at an intermediate output in the mid region between its upstream and downstream boundaries; and that the intermediate output is synchronously periodically advanced through the column of adsorbent with the feed input, raffinate output, desorbent input and extract output.

In one embodiment of the invention, the first component is para-xylene, the second component is ethylbenzene, and the third component is a mixture of meta-xylene and ortho-xylene.

In another embodiment of the invention the first component is meta-xylene, the second component is a mixture of para-xylene and ortho-xylene and the third component is ethylbenzene.

In the context of the present invention a «feed mixture» is a mixture containing one or more extract components and one or more raffinate components to be fed to an adsorbent according to the process, the term «feed stream» indicates a stream of feed mixture which passes to the adsorbent and the term «feed input» indicates the access point through which the feed stream is introduced to the adsorbent. An «extract component» is a type of compound or a compound, such as an aromatic isomer, that is more selectively adsorbed by the adsorbent while a «raffinate component» is a compound or type of compound that is less selectively adsorbed. The selectivity of the adsorbent for an «intermediate raffinate component» lies between that for the extract and raffinate components. In the first preferred embodiment of this invention, para-xylene is the extract component, and ethylbenzene, and ortho- and meta-xylene are the raffinate components. The term «raffinate output» means the withdrawal point through which a raffinate stream, i.e. a stream containing a raffinate component, is removed from an adsorbent. There are two raffinate streams in the process of this invention, the usual stream taken from the downstream boundary of the adsorption zone and an intermediate stream taken from the mid region, e.g. about the midpoint, of the adsorption zone. The composition of a raffinate stream can vary from substantially 100% desorbent to substantially 100% raffinate components. The term «extract output» is used to mean the withdrawal point through which an extract stream, i.e. a stream containing extract material which has been desorbed by a desorbent, is removed from the adsorbent. The composition of the extract stream, likewise, can vary from substantially 100% desorbent to substantially 100% extract components. Although it is possible by the process of this invention to produce high-purity extract product or raffinate product at high recoveries it will be appreciated that an extract component is never completely adsorbed by the adsorbent, nor is a raffinate component completely non-adsorbed by the adsorbent. Therefore, small amounts of a raffinate component can appear in the extract stream, and, likewise, small amounts of an extract component can appear in a raffinate stream. The extract and raffinate streams then are further distinguished from each other and from the feed mixture by the ratio of the concentrations of an extract component and a specific raffinate component, both appearing in the particular stream. For example, in the first preferred embodiment of the invention, the ratio of concentration of the more selectively adsorbed para-xylene to the concentration of less selectively adsorbed isomers and ethylbenzene will be highest in the extract stream, next highest in the feed mixture, next highest in the intermediate raffinate stream, and lowest in the raffinate stream. Likewise, the ratio of the less selectively adsorbed isomer and ethylbenzene to the more selectively adsorbed para-xylene will be highest in the raffinate stream, next highest in the intermediate raffinate stream (the only raffinate stream in which ethylbenzene is present) next highest in the feed mixture, and the lowest in the extract stream. The term «desorbent input» indicates the access point through which desorbent passes to the adsorbent. When the extract stream and the raffinate streams contain desorbent, at least a portion of the extract stream and preferably at least a portion of the raffinate streams from the adsorbent will preferably be passed to separation means, typically fractionators, for separation of at least a portion of the desorbent at separating conditions to produce an extract product and a raffinate product. The terms «extract product» and «raffinate product» mean products produced by the process containing, respectively, an extract component and a raffinate component in higher concentrations than those found in the respective extract stream and the raffinate stream.

Feed mixtures which can be utilized in the process of this invention comprise a mixture of three components such that the adsorbent and desorbent utilized exhibit relative selectivities for the three components in a descending order of magnitude. A component may comprise more than one molecular species or isomer. For example, in a preferred embodiment of the invention, the first component is para-xylene, the second component is ethylbenzene and the third component is a mixture of meta-xylene and ortho-xylene. The adsorbent-desorbent combination chosen for use in that embodiment, hereinafter discussed in greater detail, exhibits the highest relative selectivity for para-xylene, the next highest for ethylbenzene and the lowest for meta- and ortho-xylene.

To effect separation according to this invention the feed mixture is contacted with the particular adsorbent chosen and the component having the highest relative selectivity is more selectively adsorbed and is retained by the adsorbent while the other components are relatively unadsorbed and are removed from the interstitial void spaces between the particles of adsorbent and from the surface of the adsorbent. The adsorbent containing the more selectively adsorbed component is referred to as a «rich» adsorbent — i. e. rich in the more selectively adsorbed component. The adsorbed component is then recovered from the rich adsorbent by contacting the rich adsorbent with a desorbent.

The desorbents used in this process vary depending on the type of operation employed. The term «desorbent» as used herein means any fluid capable of removing a selectively adsorbed feed component from the adsorbent. In connection with the nature and function of desorbents attention is directed to the passage in UK patent publication No. 2 049 667 from page 4, line 45 to page 5, line 6, all the comments in that passage also applying to the present invention.

In the first preferred embodiment of the present invention, para-diethylbenzene is a particularly effective desorbent when used with the hereinafter described adsorbent whereas in the second preferred embodiment toluene is particularly effective in conjunction with the hereinafter described adsorbent.

The prior art has also recognized that certain characteristics of adsorbents are highly desirable, if not absolutely necessary, to the successful operation of a selective adsorption process. In this connection attention is directed to the passage in UK patent publication No. 2 049 667 from page 5 line 26 to page 6 line 61, all the comments in that passage also applying to the present invention.

The adsorbents which are used in the process of this invention will usually comprise specific crystalline aluminosilicates (molecular sieves). Details of particular crystalline aluminosilicates suitable for use in the present invention appear in UK Patent Publication No. 2 049 667 in the passage from page 6 line 63 to page 8 line 7, all the comments in that passage also applying to the present invention.

We have found that the adsorbent that has demonstrated the best selectivity for the para-xylene is an X zeolite containing barium at exchangeable cationic sites. Correspondingly, we have observed that the adsorbent that demonstrates the best selectivity for meta-xylene is a Y zeolite containing sodium at exchangeable cationic sites. Adsorbents for this process are preferably made by essentially completely ion-exchanging sodium-type X or sodium-type Y base materials, in a particle size range of from 20 to 60 U. S. mesh, with the desired cation. Typically, the ion-exchanges will be done with aqueous solutions of the soluble salts, such as the chlorides, of the respective metals. The term «essentially complete» means that the residual sodium content of the adsorbent after the ion-exchange of the base material should preferably be less than 2 wt.% $Na_2O$. After ion-exchange and water wash to remove excess ion-exchange solution the adsorbent will de dried to reduce the water content, as measured by loss on ignition (LOI) at 900°C, generally to less than 10 wt.% and more preferably to within a range of from 2 to 7 wt.%. Maintaining adsorbent water content within this preferred range has been found to maintain optimum adsorbent and process performance and water may be added to or removed from the process during operations as necessary to maintain this range. By knowing the initial water content of the adsorbent and by analyzing the process input and output streams for water content the water content of the adsorbent during process operation can be calculated. Water may be added to the adsorbent if necessary either on an intermittent or more preferably on a continuous basis by itself or in admixture with feed or desorbent material to maintain the desired concentration of water on the adsorbent. Water may be removed from the adsorbent if necessary by passing a very dry feed stream to the process and allowing the output streams to remove some water from the adsorbent until the desired range is achieved.

The adsorbent is employed in a simulated moving-bed countercurrent flow system. With the simulated moving-bed flow systems a feed mixture and a desorbent material are continuously fed to the process and adsorption and desorption are continuously taking place which allows continuous production of an extract stream and raffinate streams. The basic operating principles and sequence of operation of one such simulated moving-bed countercurrent flow system are described in U. S. Patent 2,985,589. The present invention utilizes the system described in U. S. Patent 2,985,589, to which is added the provision in this invention for withdrawal of the additional intermediate raffinate stream. In such a system it is the progressive movement of multiple liquid access and withdrawal points down an absorbent chamber that simulates the upward movement of an adsorbent contained in the chamber. Only five of the access lines are active at any one time in the present invention; the feed input, desorbent input, raffinate output, intermediate raffinate output and extract output access lines. Coincident with this simulated upward movement of a solid adsorbent is the movement of a liquid occupying the void volume of the packed bed of adsorbent. So that countercurrent contact is maintained, a liquid flow down the adsorbent chamber may be provided by a pump. As an active liquid access point moves through a cycle, that is, from the top of the chamber to the bottom, the chamber circulation pump moves through different zones which require different flow rates. A programmed flow controller may be provided to set and regulate these flow rates.

The active liquid access points effectively divide the adsorbent chamber into separate zones, each of which has a different function. In the process it is necessary that three separate operational zones be present in order for the desired operations to take place and in some instances a fourth operational zone may be present also.

The adsorption zone, hereinafter referred to as zone 1, is made up of the adsorbent located between a feed input and a raffinate output. In this zone, a feed mixture contacts an adsorbent, an extract component is adsorbed, and a raffinate stream is withdrawn at the downstream boundary of the zone. The intermediate raffinate stream is withdrawn from the adsorption zone via the intermediate raffinate output at a locus in the mid region, e. g. approximately midway, between the upstream and downstream boundaries of the zone. Since the general flow through zone 1 is from the feed input towards the raffinate outputs the flow in this zone is considered to be in a downstream direction when proceeding from the feed input to the raffinate outputs.

Immediately upstream with respect to fluid flow in zone 1 is the purification zone, hereinafter referred to as zone 2. The purification zone is made up of the adsorbent between an extract output and the feed input. The basic operations taking place in zone 2 are the displacement from the non-selective void volume (which term is defined in UK Patent Publication No. 2 049 667 and used in the same sense here) of the adsorbent of any raffinate material carried into zone 2 by the shifting of adsorbent into this zone and the desorption of any raffinate material adsorbed within the selective pore volume (likewise defined in UK Patent Publication No. 2 049 667 and used in the same sense here) of the adsorbent or adsorbed on the surfaces of the adsorbent particles. Purification is achieved by passing a portion of extract stream material leaving zone 3 (hereinafter described) into zone 2 at zone 2's upstream boundary, the extract output, to effect the displacement of raffinate material. The flow of material in zone 2 is in a downstream direction from the extract output to the feed input.

Immediately upstream of zone 2 with respect to the fluid flowing in zone 2 is the desorption zone, hereinafter referred to as zone 3. The desorption zone is made up of the adsorbent between a desorbent input and the extract output. The function of the desorption zone is to allow a desorbent which passes into this zone to displace the extract component which was adsorbed upon the adsorbent during a previous contact with feed in zone 1 in a prior cycle of operation. The flow of fluid in zone 3 is essentially in the same direction as that of zones 1 and 2.

In some instances an optional buffer zone, hereinafter referred to as zone 4, may be utilized. If used, this zone, made up of the adsorbent between the raffinate output at the downstream boundary of zone 1 and the desorbent input, is located immediately upstream, with respect to the fluid flow, of zone 3. Zone 4 would be utilized to conserve the amount of desorbent utilized in the desorption step since a portion of the raffinate stream which is removed from zone 1 can be passed into zone 4 to displace desorbent present in that zone out of that zone into the desorption zone. Zone 4 will contain enough adsorbent so that raffinate material present in the raffinate stream passing out of zone 1 and into zone 4 can be prevented from passing into zone 3 and thereby contaminating the extract stream removed from zone 3. In the instances in which the fourth operational zone is not utilized the raffinate stream passed from zone 1 to zone 4 must be carefully monitored in order that the flow directly from zone 1 to zone 3 can be stopped when there is an appreciable quantity of raffinate material present in the raffinate stream passing from zone 1 into zone 3, so that the extract outlet stream is not contaminated.

A cyclic advancement of the input and output streams through the fixed bed of an adsorbent can be accomplished by utilizing a manifold system in which the valves in the manifold are operated in a sequential manner to effect the shifting of the input and output streams thereby allowing a flow of fluid with respect to solid adsorbent in a countercurrent manner. Another mode of operation which can effect the countercurrent flow of solid adsorbent with respect to fluid involves the use of a rotating disc valve in which the input and output streams are connected to the valve and the lines through which feed, extract, desorbent and raffinate streams pass are advanced in the same direction through the adsorbent bed. Both the manifold arrangement and disc valve are known in the art. Specifically rotary disc valves which can be utilized in this operation can be found in U. S. Patents 3,040,777 and 3,422,848. Both of the aforementioned patents disclose a rotary type connection valve in which the suitable advancement of the various inputs and outputs from fixed sources can be achieved without difficulty.

In many instances, one operational zone will contain a much larger quantity of an adsorbent than some other operational zone. For instance, in some operations the buffer zone can contain a minor amount of an adsorbent as compared to the adsorbent required for the adsorption and purification zones. It can also be seen that when a very efficient desorbent material is used which can easily desorb an extract component from an adsorbent, it is possible that a relatively small amount of adsorbent will be needed in a desorption zone as compared to the adsorbent needed in the buffer zone or adsorption zone or purification zone. It is not required that an adsorbent be located in a single column which is divided into zones, and the use of multiple chambers or a series of columns is also within the scope of this embodiment.

It is not necessary that all of the inputs or outputs be simultaneously used, and in fact, in many instances some of them can be shut off while others effect an input or output of material. One apparatus which can be utilized to effect the process of this invention in a preferred embodiment contains a series of individual beds connected by connecting conduits upon which are placed input or output taps to which the various streams can be attached and alternately and periodically shifted to effect continuous operation. In some instances, the connecting conduits can be connected to transfer taps which during the normal operations function intermittently as a conduit through which material passes into or out of the process.

It is contemplated that, generally speaking, at least a portion of the extract stream will pass into a separation means wherein at least a portion of the desorbent material can be separated at separating conditions to produce an extract product containing a reduced concentration of desorbent material. Preferably, but not necessary to the operation of the process, at least a portion of each raffinate stream will also be passed to a separation means wherein at least a portion of the desorbent material can be separated at separating conditions to produce a desorbent stream which can be reused in the process and a raffinate product containing a reduced concentration of desorbent. Typically, the concentration of desorbent in the extract product and the raffinate products will be less than 5 vol.% and more preferably less than 1 vol.%. The separation means will typically be a fractionation column, the design and operation of which is well known to the separation art.

Reference can be made to U. S. Patent 2,985,589 and to a paper entitled «Continuous Adsorptive Processing - A New Separation Technique» by D. B. Broughton presented at the 34th Annual Meeting of the Society of Chemical Engineers at Tokyo, Japan on April 2, 1969, for further explanation of the simulated moving-bed countercurrent process flow scheme.

Although both liquid and vapor phase operations can be used in many adsorptive separation processes, liquid-phase operation is preferred for this process because of the lower temperature requirements and because of the higher yields of an extract product that can be obtained with liquid-phase operation over those obtained with vapor-phase operation. Effective adsorption conditions typically include a temperature of from 20°C to 250°C, with 100°C to 200°C being more preferred, and a pressure of from 1 to 36 kg/cm² (0 to 500 psig), with 1 to 19 kg/cm² (0 to 250 psig) being more preferred, to ensure liquid phase. Desorption conditions typically include the same range of temperatures and pressures as used for adsorption conditions.

The size of the units which can utilize the process of this invention can vary anywhere from those of pilot-plant scale (see for example U. S. Patent 3,706,812) to those of commercial scale and can range in flow rates from as little as a few cc an hour to many thousands of litres per hour.

The following illustrative embodiments are presentend for illustration purposes and more specifically are presented to illustrate the selectivity relationships that make the process of the invention possible. Reference to specific cations, desorbent materials, feed mixtures and operating conditions is not intended to restrict the invention to their use alone.

Illustrative embodiments

These illustrative embodiments illustrate the preferred embodiment of the present invention in which the feed stream comprises a mixture of ethylbenzene and ortho-, meta- and para-xylene.

In the first of these embodiments, the desorbent used is diethylbenzene, and the adsorbent used is X-zeolite ion-exchanged with barium as the predominant cation. The relative selectivities for this system are as follows:

|  | $\beta$ |
| --- | --- |
| para-xylene | 1.8 |
| ethylbenzene | 1.0 |
| meta-xylene | 0.5 |
| ortho-xylene | 0.5 |

In addition to the large commercial market for para-xylene, there is also a limited market for pure meta-xylene. The conventional simulated moving-bed scheme, however, in which para-xylene is the extract component, does not enable pure meta-xylene to be recovered because meta-xylene cannot be readily recovered from the raffinate by fractionation as it is inseparable from the small amounts of para-xylene that appear in the raffinate and is likewise difficult to separate from ethylbenzene. Meta-xylene, however, is readily separated by distillation from mixtures with ortho-xylene.

In view of the above, the present invention is of particular importance in connection with this embodiment, i. e. one in which para-xylene is the first component, ethylbenzene is the second component, and a mixture of meta-xylene and ortho-xylene is the third component. The first component comprises the extract stream, a mixture of the second and third components the intermediate raffinate stream, and the third component the raffinate stream. Since the meta- and ortho-xylene comprising the raffinate stream is free of para-xylene and substantially free of ethylbenzene, essentially pure meta-xylene may be recovered from the raffinate stream by simple fractionation. The intermediate raffinate stream in this embodiment may for example be passed to an isomerization unit for conversion of the ethylbenzene to xylene.

The following is a representative material balance illustrative of this embodiment of the invention.

Material Balance

| | Feed | Product | Raffinate | Intermediate Raffinate |
|---|---|---|---|---|
| Para-xylene | 20 | 19.50 | 0.00 | 0.50 |
| Meta-xylene | 45 | 0.03 | 22.97 | 22.00 |
| Ortho-xylene | 15 | 0.01 | 7.65 | 7.34 |
| Ethylbenzene | 20 | 0.07 | 0.04 | 19.89 |
| | 100 | 19.61 | 30.66 | 49.73 |

In another illustrative embodiment of the present invention in which the feed stream comprises a mixture of ethylbenzene and ortho-, meta- and para-xylenes, the desorbent used is toluene, and the adsorbent used is Y-zeolite with sodium as the predominant cation. The relative selectivities for this system are ar follows:

| | β |
|---|---|
| meta-xylene | 1.80 |
| para-xylene | 1.00 |
| ortho-xylene | 1.00 |
| ethylbenzene | 0,44 |

In this embodiment, meta-xylene is the first component, a mixture of para-xylene and ortho-xylene is the second component, and ethylbenzene is the third component. The first component. is present in the extract stream, a mixture of the second and third components is present in the intermediate raffinate stream, and the third component is present in the raffinate stream. The following is a representative material balance illustrative of this embodiment of the invention.

Material Balance

| | Feed | Extract | Raffinate | Intermediate Raffinate |
|---|---|---|---|---|
| Meta-xylene | 45 | 44.8 | 0.00 | 0.20 |
| Para-xylene | 20 | 0.1 | 0.05 | 19.85 |
| Ortho-xlene | 15 | 0.1 | 0.05 | 14.85 |
| Ethylbenzene | 20 | 0.0 | 12.00 | 8.00 |
| | 100 | 45.00 | 12.10 | 42.00 |

Since the ethylbenzene comprising the raffinate stream is free of meta-xylene and essentially pure the raffinate stream may be passed to an isomerization unit for conversion of the ethylebenzene to xylene.

Claims

1. A process for separating first, second and third components from a fluid feed stream comprising a mixture of said components employing an adsorbent and a desorbent, which process comprises the steps of:
a) maintaining net fluid flow in a single direction through a column of the adsorbent containing at least three zones having separate operational functions occurring therein and being serially interconnected to provide a continuous cycle;
b) maintaining in the column an adsorption zone comprising the adsorbent located between a feed input at its upstream boundary and a raffinate output at its downstream boundary;
c) maintaining a purification zone in the column immediately upstream from the adsorption zone and comprising the adsorbent located between an extract output at its upstream boundary and the feed input at its downstream boundary;

d) maintaining a desorption zone in the column immediately upstream from the purification zone and comprising the adsorbent located between a desorbent input at its upstream boundary and the extract output at its downstream boundary;

e) passing the feed stream via the feed input into the adsorption zone at adsorption conditions to effect the selective adsorption of the first component by the adsorbent in the adsorption zone;

f) passing a desorbent via the desorbent input into the desorption zone at desorption conditions to effect the displacement of the first component from the adsorbent in the desorption zone;

g) withdrawing an extract stream comprising the first component and desorbent material from the desorption zone via the extract output;

h) withdrawing a raffinate stream comprising a component of the fluid feed stream and desorbent material from the adsorption zone via the raffinate output; and

i) periodically advancing through the column of adsorbent, in a downstream direction with respect to fluid flow, the feed input, raffinate output, desorbent input and extract output to effect the shifting of zones through the adsorbent;

characterised in that the adsorbent and desorbent in combination exhibit relative selectivities for the first, second and third components respectively in decreasing orders of magnitude; that the withdrawn raffinate stream comprises the third component of the fluid feed stream and desorbent; that an intermediate raffinate stream comprising a mixture of the second component, the third component and desorbent is withdrawn from the adsorption zone at an intermediate output in the mid region between its upstream and downstream boundaries; and that the intermediate output is synchronously periodically advanced through the column of adsorbent with the feed input, raffinate output, desorbent input and extract output.

2. A process as claimed in claim 1 wherein the adsorption and desorption conditions include a temperature of from 20°C to 250°C and a pressure of from 1 to 36 kg/cm² (0 to 500 psig) to achieve liquid phase.

3. A process as claimed in claim 1 or 2 wherein at least portions of the raffinate stream, the extract stream and the intermediate raffinate stream are each passed to separate separation means wherein at least a portion of the desorbent is separated from them to produce raffinate, extract and intermediate raffinate products, respectively.

4. A process as claimed in any of claims 1 to 3 wherein a buffer zone is maintained in the column immediately upstream from the desorption zone and comprising the adsorbent located between the desorbent input at its downstream boundary and the raffinate output at its upstream boundary.

5. A process as claimed in any of claims 1 to 4 wherein the first component is para-xylene, the second component is ethylbenzene, and the third component is a mixture of meta-xylene and ortho-xylene.

6. A process as claimed in claim 5 wherein the adsorbent comprises an X-zeolite containing barium ions at exchangeable cationic sites and the desorbent comprises para-diethylbenzene.

7. A process as claimed in any of claims 1 to 4 wherein the first component is meta-xylene, the second component comprises a mixture of para-xylene and ortho-xylene, and the third component is ethylbenzene.

8. A process as claimed in claim 7 wherein the adsorbent comprises a Y-zeolite containing sodium ions at exchangeable cationic sites and the desorbent comprises toluene.

9. A process as claimed in claim 5 or 6 wherein the product containing meta-xylene and ortho-xylene is passed to a separation means wherein meta-xylene and ortho-xylene are separated from one another.

**Patentansprüche**

1. Verfahren zur Trennung erster, zweiter und dritter Bestandteile aus einem ein Gemisch dieser Bestandteile enthaltenden flüssigen Zulaufstrom unter Verwendung eines Adsorbens und eines Desorbens, wobei das Verfahren darin besteht, dass man stufenweise:

a) eine Nettoflüssigkeitsströmung in einer einzigen Richtung durch eine Adsorbenssäule mit mindestens drei Zonen aufrechterhält, in welchen getrennte Funktionen ablaufen und die zu einem kontinuierlichen Kreislauf hintereinandergeschaltet sind;

b) in der Säule eine Adsorptionszone aufrechterhält, die das Adsorbens zwischen einer Zulaufstelle an ihrer Anströmbegrenzung und einer Raffinatentnahme an ihrer Abströmbegrenzung angeordnet enthält;

c) in der Säule unmittelbar vor der Adsorptionszone eine Reinigungszone aufrechterhält, die das Adsorbens zwischen einer Extraktentnahme an ihrer Anströmbegrenzung und der Zulaufstelle an ihrer Abströmbegrenzung angeordnet enthält;

d) in der Säule unmittelbar vor der Reinigungszone eine Desorptionszone aufrechterhält, die das Adsorbens zwischen einem Desorbenszulauf an ihrer Anströmbegrenzung und der Extraktentnahme an ihrer Abströmbegrenzung angeordnet enthält;

e) den Zulaufstrom über die Zulaufstelle unter solchen Adsorptionsbedingungen in die Adsorptionszone leitet, dass eine selektive Adsorption des ersten Bestandteils durch das Adsorbens in der Adsorptionszone stattfindet;

f) ein Desorbens über den Desorbenszulauf unter solchen Desorptionsbedingungen in die Desorptionszone leitet, dass der erste Bestandteil aus dem Adsorbens in die Desorptionszone verdrängt wird;

g) einen den ersten Bestandteil und Desorptionsmittel enthaltenden Extraktstrom über die Extraktentnahme aus der Desorptionszone abzieht;

h) einen einen Bestandteil des flüssigen Zulaufstroms und Desorptionsmittel enthaltenden Raffinatstrom über die Raffinatennahme aus der Adsorptionszone abzieht; und

i) von Zeit zu Zeit die Zulaufstelle, die Raffinatennahme, den Desorbenszulauf und die Extraktentnahme durch die Adsorbenssäule hindurch stromabwärts bezüglich der Flüssigkeitsströmung vorrückt, um die Zonen durch das Adsorbens hindurch zu verschieben;

dadurch gekennzeichnet, dass das Adsorbens und Desorbens kombiniert relative Selektivitäten abnehmender Grössenordnung für den ersten, zweiten bzw. dritten Bestandteil aufweisen, dass der entnommene Raffinatstrom den dritten Bestandteil des flüssigen Zulaufstroms sowie Desorbens enthält, dass ein Zwischenraffinatstrom aus einem Gemisch des zweiten Bestandteils, des dritten Bestandteils und Desorbens aus der Adsorptionszone an einer Zwischenentnahme im mittleren Bereich zwischen deren Anström- und Abströmbegrenzungen abgezogen wird und dass die Zwischenentnahme von Zeit zu Zeit synchron mit der Zulaufstelle, der Raffinatennahme, dem Desorbenszulauf und der Extraktentnahme durch die Adsorbenssäule hindurch vorgerückt wird.

2. Verfahren nach Anspruch 1, worin die Adsorptions- und Desorptionsbedingungen zwecks Aufrechterhaltung einer flüssigen Phase eine Temperatur von 20°C bis 250°C und einen Druck von 1 bis 36 kg/cm² umfassen.

3. Verfahren nach Anspruch 1 oder 2, worin mindestens Anteile des Raffinatstroms, des Extraktstroms bzw. des Zwischenraffinatstroms jeweils zu getrennten Trennvorrichtungen geleitet werden, in denen mindestens ein Teil des Desorbens von diesen zur Gewinnung von Raffinat-, Extrakt- bzw. Zwischenraffinatprodukten abgetrennt wird.

4. Verfahren nach einem der Ansprüche 1 bis 3, worin in der Säule unmittelbar vor der Desorptionszone eine Pufferzone aufrechterhalten wird, die das Adsorbens zwischen dem Desorbenszulauf an ihrer Abströmbegrenzung und der Raffinatentnahme an ihrer Anströmbegrenzung angeordnet enthält.

5. Verfahren nach einem der Ansprüche 1 bis 4, worin als erster Bestandteil para-Xylol, als zweiter Bestandteil Äthylbenzol und als dritter Bestandteil ein Gemisch von meta-Xylol und ortho-Xylol vorliegt.

6. Verfahren nach Anspruch 5, worin das Adsorbens aus einem X-Zeolith mit Bariumionen an austauschbaren Kationenstellen und das Desorbens aus para-Diäthylbenzol besteht.

7. Verfahren nach einem der Ansprüche 1 bis 4, worin als erster Bestandteil meta-Xylol, als zweiter Bestandteil ein Gemisch aus para-Xylol und ortho-Xylol sowie als dritter Bestandteil Äthylbenzol vorliegt.

8. Verfahren nach Anspruch 7, worin das Adsorbens aus einem Y-Zeolith mit Natriumionen an austauschbaren Kationenstellen und das Desorbens aus Toluol besteht.

9. Verfahren nach Anspruch 5 oder 6, worin das meta-Xylol und ortho-Xylol enthaltende Produkt zu einer Trennvorrichtung geleitet wird, in welcher meta-Xylol und ortho-Xylol voneinander getrennt werden.

**Revendications**

1. Procédé pour la séparation du premier, second et troisième composants d'un courant d'alimentation fluide comprenant un mélange de ces composants en utilisant un adsorbant et un désorbant, lequel procédé comprend les stades:

a) d'entretenir un écoulement de fluide net dans une direction unique à travers une colonne de l'adsorbant contenant au moins trois zones ayant des fonctions opérationnelles distinctes qui s'y trouvent et qui sont interconnectées en série pour établir un cycle continu;

b) d'entretenir dans la colonne une zone d'adsorption comprenant l'adsorbant situé entre une admission d'alimentation à sa limite d'amont et une sortie de raffinat à sa limite d'aval;

c) d'entretenir une zone de purification dans la colonne immédiatement à l'amont de la zone d'adsorption et comprenant l'adsorbant situé entre une sortie d'extrait à sa limite d'amont et l'admission d'alimentation à sa limite d'aval;

d) d'entretenir une zone de désorption dans la colonne immédiatement à l'amont de la zone de purification et comprenant l'adsorbant situé entre une admission de désorbant à sa limite d'amont et la sortie d'extrait à sa limite d'aval;

e) de faire passer le courant d'alimentation par l'admission d'alimentation dans la zone d'adsorption dans des conditions d'adsorption pour provoquer l'adsorption sélective du premier composant par l'adsorbant dans la zone d'adsorption;

f) de faire passer un désorbant par l'admission de désorbant dans la zone de désorption dans des conditions de désorption pour provoquer le déplacement du premier composant hors de l'adsorbant dans la zone de désorption;

g) d'évacuer un courant d'extrait comprenant le premier composant et de la matière désorbante hors de la zone de désorption par la sortie d'extrait;

h) d'évacuer un courant de raffinat comprenant un composant du courant d'alimentation fluide et de la matière désorbante hors de la zone d'adsorption par la sortie de raffinat, et

i) de faire avancer périodiquement dans la colonne d'adsorbant, en direction d'aval par rapport à l'écoulement de fluide, l'admission d'alimentation, la sortie de raffinat, l'admission de désorbant et la sortie d'extrait pour provoquer le déplacement des zones dans l'adsorbant;

caractérisé en ce que l'adsorbant et le désorbant en combinaison manifestent des sélectivités pour les premier, second et troisième composants respectivement par ordre de grandeur décroissant, que le courant de raffinat évacué comprend le troisième composant du courant d'alimentation fluide et du désorbant; qu'un courant de raffinat intermédiaire comprenant un mélange du second composant, du troisième composant et de désorbant est évacué de la zone d'adsorption à une sortie intermédiaire dans la région médiane entre ses limites d'amont et d'aval et que la sortie intermédiaire est avancée périodiquement en synchronisme dans la colonne d'adsorbant avec l'admission d'alimentation, la sortie de raffinat, l'admission de désorbant et la sortie d'extrait.

2. Procédé suivant la revendication 1, dans lequel les conditions d'adsorption et de désorption comprennent une température de 20°C à 250°C et une pression de 1 à 36 kg/cm² (0 à 500 livres par pouce carré au manomètre) pour établir la phase liquide.

3. Procédé suivant la revendication 1 ou 2, dans lequel au moins des parties du courant de raffinat, du courant d'extrait et du courant de raffinat intermédiaire sont amenées chacune à des dispositifs de séparation distincts dans lesquels au moins une partie du désorbant est séparée hors d'elles pour produire du raffinat, de l'extrait et du raffinat intermédiaire, respectivement.

4. Procédé suivant l'une quelconque des revendications 1 à 3, dans lequel une zone tampon est entretenue dans la colonne immédiatement à l'amont de la zone de désorption et comprenant l'adsorbant situé entre l'admission de désorbant à sa limite d'aval et la sortie de raffinat à sa limite d'amont.

5. Procédé suivant l'une quelconque des revendications 1 à 4, dans lequel le premier composant est le para-xylène, le second composant est l'éthylbenzène et le troisième composant est un mélange de méta-xylène et d'ortho-xylène.

6. Procédé suivant la revendication 5, dans lequel l'adsorbant comprend une zéolite X contenant des ions baryum à des sites cationiques échangeables et le désorbant comprend du para-diéthylbenzène.

7. Procédé suivant l'une quelconque des revendications 1 à 4, dans lequel le premier composant est le méta-xylène, le second composant comprend un mélange de para-xylène et d'ortho-xylène et le troisième composant est l'éthylbenzène.

8. Procédé suivant la revendication 7, dans lequel l'adsorbant comprend une zéolite Y contenant des ions sodium à des sites cationiques échangeables et le désorbant comprend du toluène.

9. Procédé suivant la revendication 5 ou 6, dans lequel le produit contenant du méta-xylène et de l'ortho-xylène est amené à un dispositif de séparation dans lequel le méta-xylène et l'ortho-xylène sont séparés l'un de l'autre.